# EUROPÄISCHE PATENTANMELDUNG

(11) **EP 2 913 672 A1**
(43) Veröffentlichungstag der Anmeldung: **02.09.2015**
(21) Anmeldenummer: 15156980.3
(22) Anmeldetag: 27.02.2015
(51) Int. Cl.: G01N 33/543, G01N 33/58, G01N 33/68

(54) **Plexzitone zur Verwendung bei der Kontrolle von Zellmembran-Ionenkanalaktivität**

(30) Priorität: 27.02.2014 DE 102014002798
(71) Anmelder: Ussembayev, Yerzhan, 2628 BA Delft (NL)
(72) Erfinder: Ussembayev, Yerzhan, 2628 BA Delft (NL)
(74) Vertreter: Jones Day

(57) **Zusammenfassung**

Die vorliegende Erfindung betrifft Plexziton-Nanostrukturen für die Verwendung in einem Verfahren zur Stimulierung von Zellen, insbesondere betrifft sie Plexziton-Nanostrukturen für die Verwendung in einem Verfahren zur Steuerung von Ionenkanälen in einer Zellmembran. Ein weiterer Aspekt der vorliegenden Erfindung ist auf ein *in vitro* Verfahren zur Stimulation, Untersuchung oder Behandlung einer Zelle gerichtet.

## Beschreibung

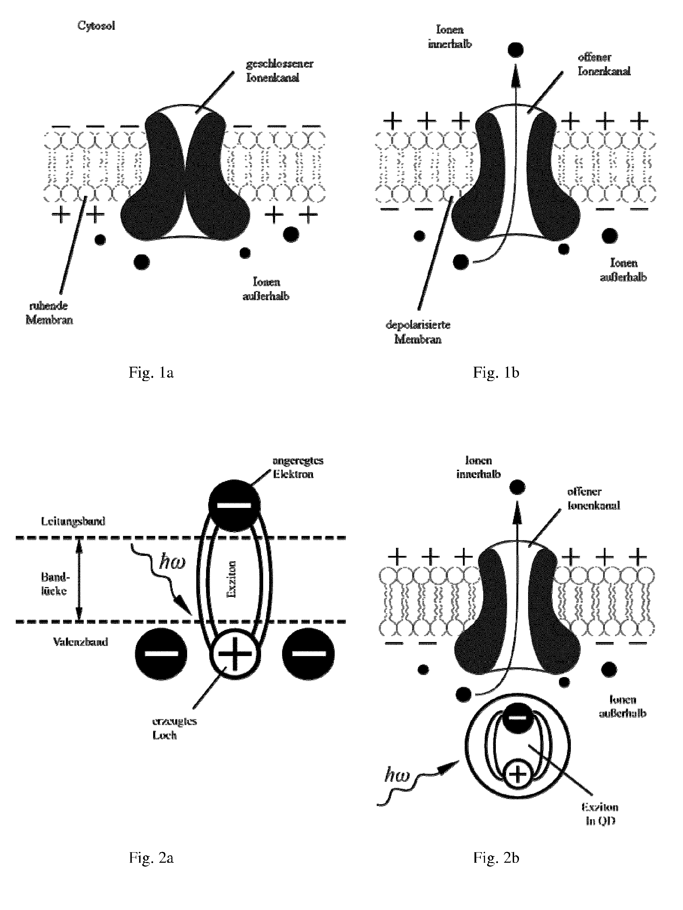

Membranionenkanäle spielen eine wichtige Rolle in der Aktivität lebender Zellen, da sie für die Kontrolle des Membranruhe- und Membranaktionspotentials, Zellvolumenregulierung als auch die Zellkommunikation und -siganlisierung von Bedeutung sind[1], [2]. Diese Ionenrezeptoren sind multimere Proteine, die in die Zellmembran eingebettet sind, wobei sie so angeordnet sind, dass sie eine Pore bilden, die sich von einer Seite des Phospholipidlayers zur anderen erstrecken (Fig. 1).

Die Ionenkanäle sind sehr empfindlich auf externe Stimulationen, wobei sie sich als Reaktion auf das erhaltene chemische, elektrische oder mechanische Signal öffnen und schließen. Abhängig vom Stimulationssignal können die Kanäle in zwei Hauptgruppen eingeteilt werden: Ionenkanal-Rezeptoren (aktiviert durch chemische Neurotransmitter) und spannungsgesteuerte Ionenkanäle (aktiviert durch elektrische Stimuli). Die letztgenannte Art von Kanälen ist für anregbare Zellen wichtig, einschließlich Nervenzellen, Berührungsrezeptoren und Muskelzellen, aufgrund ihrer Fähigkeit, ein Spannungspotential entlang der Membran zu bilden [3], [4], [5]. Insbesondere wenn die Zelle sich in einem Ruhezustand befindet, sind die Kanäle geschlossen und die Ionen (Kalium, Natrium, Calcium oder Chlorid) können nicht in das Cytosol fließen (Fig. 1a). Wenn die Zellmembran jedoch als Reaktion auf ein Stimuli depolarisiert wird, öffnen sich die Kanäle, wodurch es den Ionen in der extrazellulären Umgebung ermöglicht wird, durch die Membran in das Cytosol zu wandern (Fig. 1b). Der beschriebene Mechanismus der Ionenkanäle ist insbesondere wichtig für Nervenzellen, welche elektrische Impulse erzeugen, um die Gehirnaktivität, die Muskelkontraktion und -relaxation als auch andere biologische Prozesse zu kontrollieren. Im Allgemeinen enthalten alle bekannte Zellen Ionenkanäle, um die sie umgebende Umgebung abzutasten.

Das tiefere Verständnis von Ionenkanälen würde es erlauben, nicht nur die Aktivität der Zelle von einfachen molekularen Niveau hin zum kompletten Gehirn zu kontrollieren, sondern auch neurologische (Parkinson Erkrankung, Epilepsie) und Ionenkanal-bezogene (QT-Syndrom) Erkrankungen zu behandeln [6], [7], [8]. Deshalb haben Forscher während der letzten paar Dekaden verschiedene Strategien entwickelt, um Ionenkanäle zu aktivieren. Die am besten untersuchten Techniken sind die Patch-Clamp-Technik (das angewandte elektrische Signal depolarisiert die Zellmembran [9] und Optogenetik (genetisch modifizierte Zellen werden photoempfindlich gegenüber Lichtstimulation [10], [11]). Obwohl beide Ansätze unser neurobiologisches Wissen auf dem zellulären und dem Gesamtorganismuslevel signifikant erweitert haben, sind diese Techniken nicht ausreichend präzise für die selektive Kontrolle eines einzelnen Ionenkanals für biologische und pharmazeutische Studien. Die Entwicklung der Nanotechnologie hat jedoch neue Verfahren bereitgestellt, um dieses Problem zu lösen. Beispielsweise haben kürzlich einige Forschungsgruppen über die Schaltkontrolle von Ionenkanälen durch magnetische Nanopartikel (MNP) [12] oder Quantenpunkte (quantum dots, (QD)) [12] berichtet.

Die Aktivierung von Ionenkanälen unter Verwendung von Quantenpunkten hat große Aufmerksamkeit auf sich gezogen aufgrund der Möglichkeit, das Membranpotential einfach durch an- und ausschalten von Licht zu wechseln. Im Gegensatz zur Optogenetik ist der untersuchte Organismus nicht genetisch modifiziert, um ihn für Licht empfindlich zu machen. Das zu Grunde liegende Prinzip dieser Ionenkanal-Stimulierung ist eine Anregung von Exzitonen in QD (Fig. 2) [13]. Insbesondere wenn Licht den Halbleiter bestrahlt regt das Photon das Elektron vom Valenz- zum Leitungsband an, wobei ein positiv geladenes Loch zurückbleibt (Fig. 2, links). Aufgrund von Coulomb-Wechselwirkung zwischen dem angeregten Elektron und dem erzeugten Loch sind diese Teilchen aneinander gebunden, wobei sie ein Exziton bilden, das als ein unabhängiges Teilchen behandelt werden kann [14], [15]. Wenn der Halbleiter-QD in unmittelbare Nähe zur Zellmembran gebracht wird depolarisiert interessanterweise das angeregte Exziton die Membran, wobei die spannungsgesteuerten Ionenkanäle aktiviert werden (Fig. 2, rechts). Obwohl der entwickelte Ansatz die Möglichkeit bewiesen hat, dass die Kanäle durch Exzitonen, die bei halbleitenden Nanostrukturen induziert werden, aktiviert werden können, bleibt die Zytotoxizität von QD eine schwierige Herausforderung [16], [17], [18]. Halbleiter, wie zum Beispiel GaAs, CdSe und andere sind hoch-toxisch für die lebende Zelle und die Einkapselung dieser Teilchen in eine schützende Polymerhülle kann die Wirksamkeit der Membrandepolarisierung verringern, da QD in geringem Abstand zur Zelle platziert werden müssen.

Der vorliegenden Erfindung liegt daher die Aufgabe zugrunde, die zuvor genannten Nachteile im Stand der Technik zu überwinden. Insbesondere ist Aufgabe dieser Erfindung, Verbindungen, insbesondere im Bereich der Nanocomposites, bereitzustellen, mit der Zellen, insbesondere Zellmembrane und darin sich befindende Ionenkanäle spezifisch und gezielt stimuliert werden können ohne die Zelle negativ zu beeinflussen und/oder für diese toxisch zu sein.

Die Aufgabe wird durch die Verwendung von Plexziton-Nanostrukturen für die Verwendung in einem Verfahren zur Stimulierung von Zellen gelöst. Ein Aspekt der Erfindung ist auf eine Plexziton-Nanostruktur für die Verwendung in einem Verfahren zur Stimulation von Zellen gerichtet, insbesondere auf Plexziton-Nanostrukturen für die Verwendung in einem Verfahren zur Steuerung von Ionenkanälen in einer Zellmembran. Ein weiterer Aspekt der vorliegenden Erfindung ist auf ein *in vitro* Verfahren zur Stimulation, Untersuchung oder Behandlung einer Zelle gerichtet.

Die erfindungsgemäßen Plexziton-Nanostruktur können sowohl *in vivo* als auch *in vitro* Verfahren zur Steuerung von Ionenkanälen in einer Zellmembran verwendet werden.
Fig. 1a zeigt den Zustand einer Zelle im Ruhezustand, bei der die Ionenkanäle geschlossen sind und kein Ionen in die Zelle fließen können, während Fig. 1b den depolarisierten Zustand zeit, bei dem die Ionenkanäle offen sind und die Ionen in die Zelle fließen können.
Fig. 2a zeigt die Bildung eines Exzitons bei Bestrahlung mit Licht bestimmter Wellenlänge, während Fig. 2b die Wechselwirkung zwischen einem Exziton und einer Zelle (d.h. einem Ionenkanal) zeigt.
Fig. 3a zeigt den schematischen Aufbau eines Plexziton-Nanopartikels gemäß der vorliegenden Erfindung, bei dem AuNP (Goldnanopartikel) mit einem organischen Halbleiter (J-Aggregat oder halbleitendes Polymer) beschichtet ist; Fig. 3b zeigt die Energieänderung zwischen Plasmon- und Exziton-Zustand.
Fig. 4a zeigt die durch ein Plexziton-vermittelte Aktivierung von Membran-Ionenkanälen unter Verwendung eines LSP-unterstützten Exzitons; Fig. 4b zeigt den Zustand bei Verwendung eines schichtartigen Systems von Plexziton-Nanopartikeln.
Fig. 5a zeigt die Plexziton-vermittelte Aktivierung von Membran-Ionenkanälen unter Verwendung eines SPP-unterstützten Exzitons in einem (organischen) Halbleiterfilm, der auf einem Metallfilm (Au) in der Kretschmann-Anordnung (Prisma) abgeschieden ist; Fig. 5b zeigt die Plexziton-vermittelte Aktivierung von Membran-Ionenkanälen unter Verwendung eines SPP-unterstützten Exzitons in einem (organischen) Halbleiterfilm die auf der Schicht eines Reflexions-Diffraktionsgitter abgeschieden ist, wobei das SPP von oben oder unten angeregt werden kann.

Plexzitone gemäß der vorliegenden Erfindung sind Nanostrukturen, die ein gekoppeltes System aus Plasmonen und Exzitonen darstellen.

Kollektive Anregungen von freien Elektronen in Metallen zu Plasmaschwingungen gegen die Ionenrümpfe werden in der Festkörperphysik als Plasmonen bezeichnet. Oberflächenplasmonen (surface plasmon, SP) sind Oberflächenwellen (evaneszente Wellen), bei denen die longitudinalen elektronischen Schwingungen parallel zur Oberfläche eines Metalls angeregt werden. Die resultierende elektrische Feldstärke ist im Raum über der metallischen Oberfläche verstärkt. D.h. ein Oberflächenplasmon ist eine elektromagnetische Anregung, die durch kollektive Oszillierung von freien Elektronen in einem Metall, das durch einfallendes Licht, welches das Metall-dielektrische Interface bestrahlt, angeregt wird. Es gibt zwei Arten von Oberflächenplasmonen, abhängig von der Geometrie der Nanostruktur. Beispielsweise, wenn die einfallende elektromagnetische Welle Nanopartikel eines edlen Metalls (wie zum Beispiel Silber oder Gold) bestrahlt, werden kollektive kohärente Oszillationen von freien Elektronen auf der Oberfläche des Metalls erzeugt. Diese Elektronenoszillationen verursachen eine Ladungstrennung, wodurch kohärente Dipoloszillationen - "localized surface plasmon" (LSP) genannt - gebildet werden. Bei einer spezifischen Licht-Resonanzfrequenz kann die kollektive Elektronenoszillation, die auf das Teilchen beschränkt ist, eine maximale Amplitude erreichen, was in einer großen Verstärkung eines einfallenden elektromagnetischen Felds resultiert [21].

Dieses Phänomen ändert sich, wenn man ein erstrecktes dielektrisches Metallinterface, wie zum Beispiel Bulkmetall oder eine dünne Folie verwendet. Bei dieser Art von Strukturen sind die kohärenten Dipoloszillationen nicht auf die Geometrie im Nanomaßstab beschränkt und sie propagieren entlang der Metalloberfläche. Diese propagierende Plasmonwellen werden "surface plasmon polaritons" (SPP) genannt. Löst man die Maxwell Gleichung für das gezeigte dielektrische Metallinterface, erhält man eine Dispersionsbeziehung, aus der offensichtlich wird, dass SPP reine aperiodisch abklingende Wellen sind und aufgrund des Gesetzes der Impulserhaltung nicht direkt mit dem propagierenden Licht gekoppelt werden können [22]. Es gibt jedoch verschiedene Techniken, die es ermöglichen das auftretende Impulsungleichgewicht zu kompensieren. Beispielsweise kann dies durch die sogenannte Kretschmann-Konfiguration erreicht werden, bei der der SPP-Wellenvektor an die abklingende Welle gekoppelt wird, was mit einer vollständigen internen Reflexion des Laserstrahls in dem Prisma verbunden ist. Alternativ kann die SPP auf dem Gitter oder einigen kleinen Nanomerkmalen angeregt werden, wobei der SPP-Impuls mit den übereinstimmenden Komponenten des gebeugten Lichts gekoppelt ist.

Beide der beschriebenen Oberflächenplasmonen haben eine einzigartige Eigenschaft - Verstärkung oder Feldüberhöhung des lokalen elektromagnetischen Felds. Diese Merkmal macht Plasmonen für verschiedene Anwendungsgebiete interessant, einschließlich optischer Telekommunikation (plasmonische Wellenleiter und Filter [23]), Biosensorik (SERS - oberflächenverstärkte Ramanspektroskopie [24], [25]) und die Biomedizin (Goldnanoteilchen und Nanostäbe für photodynamische Krebstherapie [26], [27]) und Kontrastmittel für bildgebende Verfahren [28]). Deshalb haben Plasmonen ein vielversprechendes Potential, um ebenfalls für Membranionenkanal-Studien verwendet zu werden, da ihre Eigenschaften präzise durch die Anregungswellenlänge, angewandte dielektrische Materialien und verschiedene Größen und Geometrie der entworfenen Nanostrukturen kontrolliert werden können.

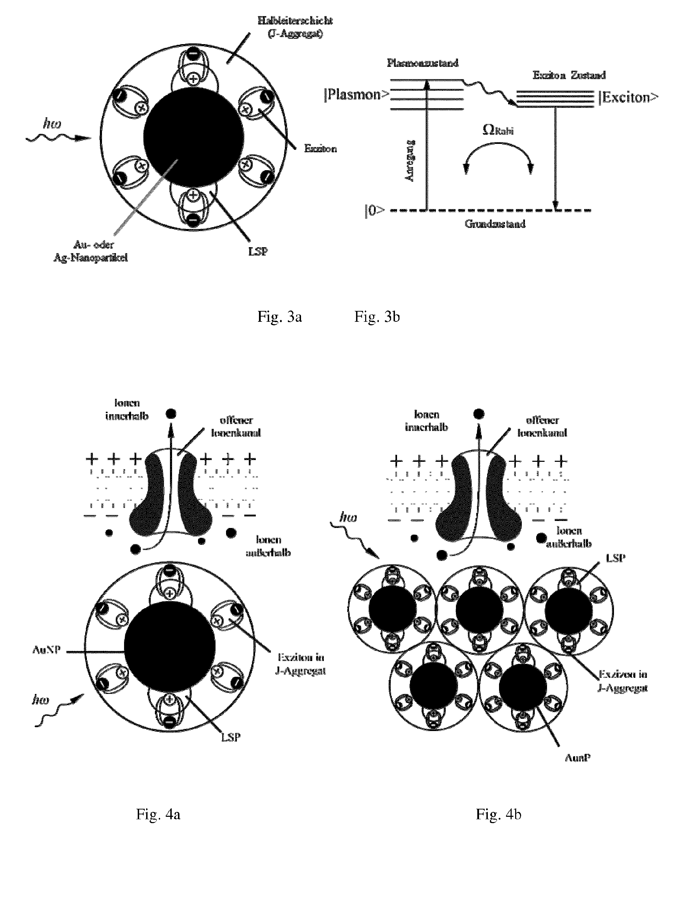

Die oben genannten Eigenschaften der Plasmonen werden mit exzitonischen Eigenschaften gekoppelt, um die Plexzitonen der Erfindung zu erhalten, d.h. es kommt zu einer Wechselwirkung zwischen Plasmonen mit Exzitonen. Werden beispielsweise Metallnanoteilchen (MNP) mit einer dünnen Schicht eines organischen Halbleiters, wie zum Beispiel J-Aggregate oder halbleitende Polymere oder eines der unten genannten Materialien, beschichtet (Fig. 3a), transferiert das induzierte LSP seine Energie zu dem Exziton, das in dem umgebenden Film angeregt wird (Fig. 3b). Innerhalb dieser Wechselwirkung oszilliert die Energie mit Rabi-Frequenz zwischen den Zuständen des LSP und Exzitons, wobei die Wirksamkeit und Stabilität des induzierten Plexziton-Systems erhöht wird [29], [30]. Diese Art der Plasmon-Exziton Wechselwirkung kann für plasmonische sphärische Teilchen, als auch für dünne Metallfilme [32], [33], Schichtsysteme von Nanopartikeln [34], [35], periodische Anordnungen von Nanostäbchen [36], Nanoporen [37] und Schlitze [38] und viele andere nanostrukturierten Geometrien, bei denen LSP als auch SPP Moden wirksam mit Exzitonen gekoppelt werden können, eingesetzt werden

Die Plexziton-Nanoteilchen, die in der vorliegenden Erfindung zum Einsatz kommen, sind aus einem Kern aus einem Metall-Nanoteilchen und einer den Kern umhüllenden Schicht aufgebaut.

Beispiele solcher Kern-Matelialien sind Metalle und deren Verbindungen. In einer Ausführungsform der vorliegenden Erfindung wird das Kern-Material aus Metallen, wie zum Beispiel Au, Ag, Cu, Al, Pt oder Pd gebildet. In einer weiteren Ausführungsform ist das Kern-Material ein Edelmetall und/oder dessen Verbindungen, beispielsweise ist das Kern-Material Au und Ag. In einer Ausführungsform der Erfindung ist das Kern-Material Au. Prinzipiell hängt die Auswahl des Kernmetalls oder dessen Verbindungen davon ab, inwieweit das Metall oder dessen Verbindungen für die Zelle des Subjekts nicht oder möglichst nur geringfügig toxisch ist.

Das oben genannte Kern-Material ist von einer den Kern umhüllenden Schicht umgeben. Das Material der Schicht hat exzitonische Eigenschaften, wie oben beschrieben. Die Schicht kann beispielsweise ein organisches Halbleitermaterial, ein anorganisches Halbleitermaterial oder Cluster-Farbmoleküle sein. Generell kann jedes bekannte Material, das die oben genannten exzitonischen Eigenschaften besitzt und/oder das im Stand der Technik bekannt ist, als Schichtmaterial eingesetzt werden. Beispielsweise sind J-Aggregate, bei denen die Cluster der Farbmoleküle halbleitende Übergänge untereinander bilden, für die Verwendung in den erfindungsgemäßen Plexzitons geeignet. Beispiele für verwendbare Verbindungen sind, ohne darauf beschränkt zu sein, Perylen-bis(phenethylimide) (PPEI), Poly(vinylpyridin) (PVP), Rhodamin-6G-Farbstoff (R6G), alternativ in einer Polymermatrix wie PVA, JC1-Komplexe, natürliche molekulare Aggregate wie zum Beispiel Bchl, FMO Proteinkomplexe, Cyanin-Farbstoffe, alternativ in einer Polymermatrix, 2,2'-Dimethyl-8-phenyl-5,6,5',6'-dibenzothiacarbocyaninchloride oder 5,5',6,6'-Tetrachloro-di-(4-sulfobutyl)benzimidazolocarbocyanin.

Das Schichtmaterial kann als halbleitende Hülle an das Kern-Material gebunden sein. Jedes bekannte Verfahren kann verwendet werden, um die umhüllende Schicht an das Kern-Material zu binden. Beispielsweise kann das Schichtmaterial über eine direkte Bindung oder über chemische Absorption an den Metallkern gebunden werden. Beispielsweise kann das Kern-Metall derivatisiert werden, um darauf stabile Verknüpfungspunkte anzuordnen an die das Schichtmaterial binden kann [19], [20].

Eine wichtiges Kriterium hinsichtlich der Auswahl der Materialien für die erfindungsgemäßen Plexziton-Nanostrukturen ist, dass diese für die lebende Zelle nicht oder möglichst geringfügig toxisch sein dürfen. Die für die jeweilige Anwendung geeigneten Materialien werden dem Fachmann auf diesem Gebiet bekannt sein und somit ist die Auswahl dieser für den Fachmann jederzeit möglich.

Die erfindungsgemäße Plexziton-Nanostruktur kann ebenfalls ein Metallfilm oder - (beugungs)gitter oder jede andere periodisch angeordnete Geometrie oder (Nano-)Struktur im Nanomaßstab sein, auf dem das exzitonische Material aufgebracht ist. Beispiele solcher Geometrien sind Nanopartikel, Nanoteilchen, Nanostäbchen, Nanodrähte, Nanoporen oder Nanoschlitze. Generell ist der Begriff Plexziton-Nanostrukturen nicht auf eines bestimmte Geometrie oder Form beschränkt, sondern umfasst ebenfalls die oben genannten Geometrien und Formen.

Das Kern-Material wird in einer Größenordnung verwendet, die für den angewendeten Zweck geeignet ist. D.h. das Kern-Matelial liegt in einer Größenordnung vor, damit die Plexziton-Nanostrukturen, d.h. das Kern-Material und die dieses umhüllende Schicht zusammen eine Größenordnung ergeben, die für die Verwendung zur Beeinflussung der Funktionen einer Zelle geeignet sind. Dementsprechend ist das Verhältnis von Größe des Kerns und der umhüllenden Schicht entsprechend aufeinander abgestimmt. Die Größe des Plexziton-Nanostrukturen sollte auch so gewählt werden, dass dessen Verabreichung an das zu untersuchende oder zu behandelnde Subjekt auf die gewünschte Art und Weise möglich ist.

In einer Ausführungsform der Erfindung hat das Kern-Material eine Größe von 10 nm bis 500 nm, beispielsweise 10 nm bis 400 nm, 10 nm bis 300 nm oder 10 nm bis 250 nm. In einer anderen Ausführungsform der Erfindung hast das Kern-Matelial eine Größe von 50 nm bis 500 nm, beispielsweise 100 nm bis 500nm oder 150 nm bis 500 nm. Die untere Grenze der Größe des Kern-Materials kann 10 nm, 15 nm, 20 nm, 25 nm, 30 nm, 35 nm, 40 nm, 45 nm oder 50 nm sein, während die obere Grenze des Kern-Materials 450 nm, 460 nm, 470 n" 480 nm, 490 nm oder 500 nm sein kann.

Die Dicke des Schichtmaterials kann in einem Bereich von 10 nm bis 200 nm sein, beispielsweise in einem Bereich von 20 nm bis 100 nm. In einer Ausführungsform ist die Dicke des Schichtmaterials 50 nm.

Die primäre Lichtquelle, die für die SP Anregung verwendet wird, ist eine Laseremission. Beispielsweise können Nd:YAG Laser (1064 und 532 nm), Ti:Saphir Laser (670-1130 nm), He-Ne Laser (543-1152 nm), Rubinlaser (649 nm), Argon Laser (488-514 nm) oder Alexandrit Laser (700-800 nm). Die Anregung kann gepulst oder eine kontinuierlich Welle sein; Plasmon sind durch beide Arten von Strahlung anregbar. Neben der genannten Anregung durch Laseremission sind weitere Lichtquellen für die Anregung verwendbar, beispielsweise UV-Strahlung, sichtbares Licht, IR-Strahlung, Röntgenstrahlung, Elektronenstrahlung, phosphoreszierende Verbindungen, chemilumineszente Verbindung, biolumineszente Verbindungen oder Licht-emittierende Enzyme, ohne darauf beschränkt zu sein.

Durch Anregung unter Verwendung der geeigneten Lichtquelle wird das System Plasmon-Exziton, d.h. das Plexziton, gezielt aktiviert. Durch die Kopplung mit einem Plasmon wird die normalerweise kurze Lebenszeit eines einzelnen Exzitons verlängert und somit kann eine kontrollierte Erzeugung des Dipolmoments des Plexzitons erreicht werden. Wir die Lichtquelle abgeschaltet wird entsprechend die Anregungsenergie entfernt und das Dipolmoment (d.h. das Elektron-Loch Systems des Plexzitons) verschwindet. Die vorher angeregte Zelle kann in ihren Ursprungs- bzw. Normalzustand zurückkehren.

Die Anregungsenergie kann durch das Subjekt dringen und so die Plexziton-Nanostrukturen anregen, d.h. jede gewünschte Tiefe hinsichtlich der Durchdringung kann erreicht werden, um die Plexziton-Nanostrukturen anzuregen und die gewünschte Wirkung in der Zelle zu erzielen.

Die Größe der Teilchen des Kern-Matelials oder die Periodizität der periodisch angeordneten Nanostrukturen hat auch einen Einfluss auf die Anregungswellenlänge des SP. Beispielsweise kann, abhängig vom verwendeten Metall, für kleine Nanoteilchen im Bereich von 10 nm bis 100 nm das LSP bei einer Resonanzwellenlänge von 400-530 nm angeregt werden, während sich bei größeren Größen (oberhalb 100 nm) die Resonanzwellenlänge in den nahen Infrarotbereich verschiebt (Richtung 600nm und mehr). Die Form der Teilchen kann ebenfalls einen Einfluss auf die Resonanzwellenlänge haben. Beispielsweise können Nanodrähte oder Nanostäbchen Plasmon bei einer Wellenlänge von 750-850nm unterstützen. Ebenfalls ein zu beachtender Parameter ist die Geometrie der Anordnungen, da das Licht mit den SPP Moden über die Diffraktion gekoppelt ist, welche wiederum von der Wellenlänge und dem Einfallswinkel abhängt. Die Wellenlängen können deshalb auf einen Bereich zwischen 550nm und 1050nm eingestellt werden, bei entsprechend optimierten Einfallswinkeln für die jeweils gegebene Periodizität.

Die Plexziton-Nanostrukturen der Erfindung können in Verfahren zur Stimulation von Zellen verwendet werden. Das zu untersuchende oder zu behandelnde Subjekt ist dabei ein Säugetier. In einer Ausführungsform der Erfindung ist das Subjekt ein Mensch.

Die oben genannten Materialien können für die Plexziton-vermittelte Stimulation von Zellen, beispielsweise der Stimulation einer Ionenkanalmembran, auf verschiedene Art und Weise verwendet werden. Beispielsweise ist es bei der Verwendung zur Stimulation von Ionenkanälen, aber auch jeder anderen Art der Stimulation einer Zelle, der erste Ansatz, die Plexziton-Nanostruktur in der unmittelbaren Nähe der Zellmembran zu platzieren. Die folgende Lichtbestrahlung des Teilchens regt das Plasmon an, welches die Energie zu den Exzitonen in der Hülle transferiert. Als ein Ergebnis depolarisieren die durch Exzitoninduzierten Dipolmomente der Plexziton-Nanostrukturen die Zellmembran, was zur Aktivierung (Öffnung oder Schließung) des Ionenkanals führt. Die zu untersuchenden oder zu stimulierenden Zellen können ebenfalls gezüchtet werden und auf der Oberfläche eines geschichteten Plexziton-Nanopartikels, bei dem die einzelnen Nanoteilchen aufeinander abgeschieden werden, untersucht werden.

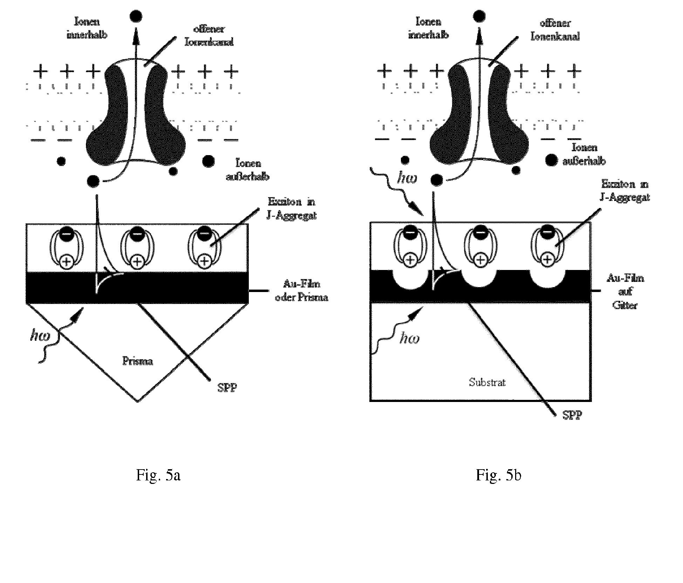

Eine andere Strategie ist es, die zu untersuchenden Zellen auf der Oberfläche eines Schichtmaterials, wie beispielsweise einen organischen halbleitenden Film oder eine J-Aggregatpolymerschicht, die durch Spin-Coating auf die Oberfläche eines Metallfilms in der Kretschmann Geometrie (Fig. 5a, ähnlich zu [32], [33]) oder Diffraktionsgitters (Fig. 5b, [38]) erhalten werden, zu säen. Bei beiden Techniken sind die Exzitonen mit den sich ausbreitenden Oberflächenplasmonmoden (SPP) gekoppelt. Diese Verfahren erfordern eine präzise Kontrolle des Einfallswinkels und -wellenlänge der Laseranregung, welche das Gesetz der Impulserhaltung genügen sollte. Sobald SPP erfolgreich mit dem einfallenden Licht gekoppelt ist, wird es die Energie zu den Exzitonen transferieren, welche wiederum die Ionenkanäle der Zelle aktivieren. SPP-Exziton Wechselwirkung wird sich entlang der Metalloberfläche ausbreiten, wobei die Ionenkanäle, die sich entlang des Weges befinden, aktiviert werden. Als ein Ergebnis kann man beispielsweise präzise Nervenzellen studieren, welche lange Axone haben (können mehrere Meter innerhalb des menschlichen Körpers betragen) und die die elektrischen Signale in entfernte Gewebestellen leiten.

Ein weiterer Aspekt der vorliegenden Erfindung ist auf die *in vitro* Verwendung der Plexziton-Nanostruktur der Erfindung gerichtet. Eine Ausführungsform der Erfindung ist daher auf ein *in vitro* Verfahren zur Stimulation, Untersuchung oder Behandlung einer Zelle, umfassend das in-Kontakt-bringen einer Plexziton-Nanostruktur mit der Zelle; und das Anwenden von Anregungsenergie in Form von Licht auf die Zelle, gerichtet.

Mittels dieses Verfahrens kann das biochemische Verhalten von Zellen untersucht werden und deren Verhalten, beispielsweise das Funktionieren von Ionenkanälen und deren kritische Parameter, offengelegt werden. Durch die durch die vorliegende Erfindung möglich gemachte gezielte Ansteuerung einzelner Zellen und Zellgruppen kann deren Verhalten und Reaktion auf externe Stimuli untersucht werden.

In Zusammenhang mit dem oben genannten *in vitro* Verfahren bedeute In-Kontakt-bringen, dass die Plexziton-Nanostrukturen in die unmittelbare Nähe der Zelle gebracht wird. Dies kann auf jede bekannte Art und Weise passieren. Beispielsweise können die Plexziton-Nanostrukturen in die Nähe der Zelle injiziert werden. Das anzuwendende Verfahren wird entsprechende den Anforderungen vom Fachmann ausgewählt und verwendet werden.

Durch die Verwendung von Plexziton-Nanostrukturen gemäß der Erfindung können Erkrankungen, insbesondere neurodegenerative Erkrankungen behandelt werden. Beispiel solcher neurodegenerativer Erkrankungen sind, ohne darauf beschränkt zu sein, Parkinson, Epilepsie, Demenz, Morbus Pick, Neuronal intermediate filament inclusion disease (NIFID), Long-QT und Huntington.

Die Plexziton-Nanostrukturen der Erfindung können alleine oder in Kombination mit anderen geeigneten Verbindungen verwendet werden. Durch die Verwendung in Kombination mit anderen Verbindungen kann eine weitere Spezifizierung erreicht werden und Krankheiten effizienter untersucht und/oder behandelt werden.

Die beschriebenen Verfahren der Plexziton-vermittelten Ionenkanal-Aktivierung haben mehrere Vorteile gegenüber den entwickelte herkömmlichen Techniken:
Die angewendeten Materialien (beispielsweise Gold und organische Exziton-Materialien) sind relativ biokompatibel, was sicherstellt, das die untersuchten Zellen unbeschädigt bleiben im Vergleich zur Intoxikation, wie sie für QD beobachtet wird.

Die Fernsteuerung wird durch Licht ohne optogene Modifikationen der Zellen unter Verwendung von Viren, die als vierte Stufe der biologischen Gefährdung angesehen werden, durchgeführt. Zudem ist die Verwendung der Plexziton nicht invasiv.

Die Größe der angewandten Nanostrukturen kann mehrere Nanometer sein, was die Präzision der Untersuchungen auf einfachem molekularen Niveau verbessert.

SPP-Exziton-Wellenführung ermöglicht die Aktivierung der Ionenkanäle, die sich innerhalb der Länge den Nervenzellen-Axone, die eine wichtige Rolle bei der Signalübertragung zu entfernten Stellen spielen, befinden.

### Referenzen:

[1] W. A. Catrerall, "Structure and Function of Voltage···Sensitive Ion Channels," Science (80···. )., vol. 242, pp. 50-61, 1988. alternatively some description in given at
[2] D. C. Gadsby, "Ion channels versus ion pumps: the principal difference, in principle.," Nat. Rev. Mol. Cell Biol., vol. 10, no. 5, pp. 344-52, May 2009.
[3] F. Tombola, M. M. Pathak, and E. Y. Isacoff, "How does voltage open an ion channel?," Annu. Rev. Cell Dev. Biol., vol. 22, pp. 23-52, Jan. 2006.
[4] K. J. Swartz, "Sensing voltage across lipid membranes.," Nature, vol. 456, no. 7224, pp. 891-7, Dec. 2008.
[5] F. Bezanilla, "How membrane proteins sense voltage.," Nat. Rev. Mol. Cell Biol., vol. 9, no. 4, pp. 323-32, Apr. 2008.
[6] S. Hatta, J. Sakamoto, and Y. Horio, "Ion channels and diseases.," Med. Electron Microsc., vol. 35, no. 3, pp. 117-26, Sep. 2002.
[7] F. Lehmann···Horn and K. Jurkat···Rott, "Voltage···gated ion channels and hereditary disease.," Physiol. Rev., vol. 79, no. 4, pp. 1317-72, Oct. 1999.
[8] S. M. Modell and M. H. Lehmann, "The long QT syndrome family of cardiac ion channelopathies: A HuGE review*," Genet. Med., vol. 8, no. 3, pp. 143-155, Mar. 2006.
[9] E. Neher and B. Sakmann, "The patch clamp technique.," Sci. Am., vol. 266, no. 3, pp. 44-51, Mar. 1992.
[10] K. Deisseroth, "Optogenetics," Nat. Methods, vol. 8, no. 1, pp. 26-29, 2011.
[11] K. Deisseroth, "Controlling the Brain with Light," Sci. Am., no. November, pp. 48-55,2010.
[12] J. Dobson, "Remote control of cellular behaviour with magnetic nanoparticles.," Nat. Nanotechnol., vol. 3, no. 3, pp. 139-43, Mar. 2008.
[13] K. Lugo, X. Miao, F. Rieke, and L. Y. Lin, "Remote switching of cellular activity and cell signaling using light in conjunction with quantum dots.," Biomed. Opt. Express, vol. 3, no. 3, pp. 447-54, Mar. 2012.
[14] W. Y. Liang, "Excitons," vol. 226, 1970.
[15] S. K. Saikin, A. Eisfeld, S. Valleau, and A. Aspuru···Guzik, "Photonics meets excitonics: natural and artificial molecular aggregates," Nanophotonics, vol. 2, no. 1, p. 17, Mar. 2013.
[16] M. Bottrill and M. Green, "Some aspects of quantum dot toxicity.," Chem. Commun. (Camb)., vol. 47, no. 25, pp. 7039-50, Jul. 2011.
[17] I. L. Medintz, H. T. Uyeda, E. R. Goldman, and H. Mattoussi, "Quantum dot bioconjugates for imaging, labelling and sensing.," Nat. Mater., vol. 4, no. 6, pp. 435-46, Jun. 2005.
[18] A. M. Derfus, W. C. W. Chan, and S. N. Bhatia, "Probing the Cytotoxicity of Semiconductor Quantum Dots," Nano Lett., vol. 4, no. 1, pp. 11-18, Jan. 2004.
[19] D. Melnikau, D. Savateeva, A. Susha, A. L. Rogach und Y. P. Rakovich, "Strong Plasmon-exciton coupling in a hybrid system of gold nanostars and J-aggregates", Nanoscale Research Letters 2013, 8:134.
[20] P. Vasa, W. Wang, R. Pomraenke, M. Lammers, M. Maiuri, C. Manzoni, G. Cerullo und C. Lienau, "Real-time observation of ultrafast Ravi oscillations between excitons and plasmons in metal nanostructures with J-aggregates", Nature Photonics, 2013, vol. 7, pp 128-132.
[21] S. Lal, S. Link, and N. J. Halas, "Nano···optics from sensing to waveguiding," Nat. Photonics, vol. 1, no. 11, pp. 641-648, Nov. 2007.
[22] A. Zayats, I. Smolyaninov, and A. Maradudin, "Nano···optics of surface plasmon polaritons," Phys. Rep., vol. 408, pp. 131-314,2005.
[23] D. K. Gramotnev and S. I. Bozhevolnyi, "Plasmonics beyond the diffraction limit," Nat. Photonics, vol. 4, no. 2, pp. 83-91, Jan. 2010.
[24] K. Hering, D. Cialla, K. Ackermann, T. Dörfer, R. Möller, H. Schneidewind, R. Mattheis, W. Fritzsche, P. Rösch, and J. Popp, "SERS: a versatile tool in chemical and biochemical diagnostics.," Anal. Bioanal. Chem., vol. 390, no. 1, pp. 113-24, Jan. 2008.
[25] J. N. Anker, W. P. Hall, O. Lyandres, N. C. Shah, J. Zhao, and R. P. Van Duyne, "Biosensing with plasmonic nanosensors.," Nat. Mater., vol. 7, no. 6, pp. 442-53, Jun. 2008.
[26] S. Lal, S. E. Clare, and N. J. Halas, "Nanoshell···Enabled Photothermal Cancer Therapy: Impending Clinical Impact," Acc. Chem. Res., vol. 41, no. 12, 2008.
[27] R. Vankayala, Y.···K. Huang, P. Kalluru, C.···S. Chiang, and K. C. Hwang, "First Demonstration of Gold Nanorods···Mediated Photodynamic Therapeutic Destruction of Tumors via Near Infra···Red Light Activation.," Small, pp. 1-11, Dec. 2013.
[28] R. Bardhan, S. Lal, A. Joshi, and N. J. Halas, "Theranostic Nanoshells: From Probe Design to Imaging and Treatment of Cancer," Acc. Chem. Res., vol. 44, no. 10, 2011.
[29] A. E. Schlather, N. Large, A. S. Urban, P. Nordlander, and N. J. Halas, "Near···field mediated plexcitonic coupling and giant rabi splitting in individual metallic dimers.," Nano Lett., vol. 13, no. 7, pp. 3281-6, Jul. 2013.
[30] Manjavacas, F. J. Garcia de Abajo, and P. Nordlander, "Quantum plexcitonics: strongly interacting plasmons and excitons.," Nano Lett., vol. 11, no. 6, pp. 2318-23, Jun. 2011.
[31] N. T. Fofang, T.···H. Park, O. Neumann, N. a Mirin, P. Nordlander, and N. J. Halas, "Plexcitonic nanoparticles: plasmon···exciton coupling in nanoshell···J···aggregate complexes.," Nano Lett., vol. 8, no. 10, pp. 3481-7, Oct. 2008.
[32] J. Bellessa, C. Bonnand, J. Plenet, and J. Mugnier, "Strong Coupling between Surface Plasmons and Excitons in an Organic Semiconductor," Phys. Rev. Lett., vol. 93, no. 3, p. 036404, Jul. 2004.
[33] C. Bonnand, J. Bellessa, and J. Plenet, "Properties of surface plasmons strongly coupled to excitons in an organic semiconductor near a metallic surface," Phys. Rev. B, vol. 73, no. 24, p. 245330, Jun. 2006.
[34] B. G. Delacy, W. Qiu, M. Solja, C. W. Hsu, O. D. Miller, S. G. Johnson, and J. D. Joannopoulos, "Layer···by···layer self···assembly of plexcitonic nanoparticles," Opt. Express, vol. 21, no. 16, pp. 4536-4537,2013.
[35] T. Ozel, P. L. Hernandez···Martinez, E. Mutlugun, O. Akin, S. Nizamoglu, I. O. Ozel, Q. Zhang, Q. Xiong, and H. V. Demir, "Observation of Selective Plasmon···Exciton Coupling in Nonradiative Energy Transfer: Donor···Selective versus Acceptor···Selective Plexcitons.," Nano Lett., vol. 13, no. 7, pp. 3065-72, Jul. 2013.
[36] S. R. K. Rodriguez and J. G. Rivas, "Surface lattice resonances strongly coupled to Rhodamine 6G excitons: tuning the plasmon···exciton···polariton mass and composition," Opt. Express, vol. 21, no. 22, p. 27411, Nov. 2013.
[37] J. Dintinger, S. Klein, F. Bustos, W. Barnes, and T. Ebbesen, "Strong coupling between surface plasmon···polaritons and organic molecules in subwavelength hole arrays," Phys. Rev. B, vol. 71, no. 3, p. 035424, Jan. 2005.
[38] P. Vasa, W. Wang, R. Pomraenke, M. Lammers, M. Maiuri, C. Manzoni, G. Cerullo, and C. Lienau, "Real···time observation of ultrafast Rabi oscillations between excitons and plasmons in metal nanostructures with J···aggregates," Nat. Photonics, vol. 7, no. February, pp. 1-5, 2013.

## Patentansprüche

1. Plexziton-Nanostruktur für die Verwendung in einem Verfahren zur Steuerung von Ionenkanälen in einer Zellmembran.

2. Plexziton-Nanostruktur für die Verwendung nach Anspruch 1, wobei die Steuerung ein Öffnen und Schließen der Ionenkanäle bewirkt.

3. Plexziton-Nanostruktur für die Verwendung nach einem der vorhergehenden Ansprüche, wobei das Plexziton ein gekoppeltes System aus einem Plasmon und einem Exziton ist.

4. Plexziton-Nanostruktur für die Verwendung nach einem der vorhergehenden Ansprüche, wobei die Plexziton-Nanostruktur aus einem Metall-Nanopartikel und einer umhüllenden Schicht aufgebaut ist.

5. Plexziton-Nanostruktur für die Verwendung nach Anspruch 4, wobei die Metall-Nanostruktur aus Ag oder Au ist.

6. Plexziton-Nanostruktur für die Verwendung nach Anspruch 4, wobei die umhüllende Schicht ein organische Halbleitermaterial, ein anorganisches Halbleitermaterial oder Cluster-Farbmoleküle ist.

7. Plexziton-Nanostruktur für die Verwendung nach einem der vorhergehenden Anspruch, wobei das Verfahren zur Behandlung einer neurodegenerativen Erkrankung verwendet wird.

8. Plexziton-Nanostruktur nach Anspruch 7, wobei die Erkrankung aus Parkinson, Epilepsie, Demenz und Long QT ausgewählt wird.

9. *In vitro* Verfahren zur Stimulation, Untersuchung oder Behandlung einer Zelle, umfassend:
• In-Kontakt-bringen einer Plexziton-Nanostruktur mit der Zelle; und
• Anwenden von Anregungsenergie auf die Zelle.

10. *In vitro* Verfahren nach Anspruch 9, wobei ein Ionenkanal in der Zelle stimuliert, untersucht oder behandelt wird.
